(19) Europäisches Patentamt
European Patent Office
Office européen des brevets

(11) **EP 4 367 502 B1**

(12) **EUROPEAN PATENT SPECIFICATION**

(45) Date of publication and mention
of the grant of the patent:
**02.07.2025 Bulletin 2025/27**

(21) Application number: **22737498.0**

(22) Date of filing: **05.07.2022**

(51) International Patent Classification (IPC):
**G01N 21/25** *(2006.01)*    **G01N 22/00** *(2006.01)*
**A23L 5/10** *(2016.01)*    **A47J 37/12** *(2006.01)*
**G01N 33/03** *(2006.01)*

(52) Cooperative Patent Classification (CPC):
**A23L 5/11; A47J 37/1214; A47J 37/1266;
G01N 22/00; G01N 33/03**

(86) International application number:
**PCT/EP2022/068588**

(87) International publication number:
**WO 2023/280853 (12.01.2023 Gazette 2023/02)**

(54) **FRYING OIL SENSING MEANS AND FRYING OIL MANAGEMENT WITHIN AN INDUSTRIAL FRYER SETUP**

FRITTIERÖLERFASSUNGSMITTEL UND FRITTIERÖLVERWALTUNG IN EINER EINRICHTUNG FÜR EINE INDUSTRIELLE FRITTIERE

MOYEN DE DÉTECTION D'HUILE DE FRITURE ET GESTION D'HUILE DE FRITURE DANS UNE INSTALLATION DE FRITEUSE INDUSTRIELLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.07.2021 EP 21184156**

(43) Date of publication of application:
**15.05.2024 Bulletin 2024/20**

(73) Proprietor: **GEA Food Solutions Bakel B.V.
5761 EN Bakel (NL)**

(72) Inventors:
• **VAN ERP, Joost
  5674 RS Nuenen (NL)**
• **VAN LANKVELD, Luuc
  5761 CT Bakel (NL)**

(74) Representative: **Kutzenberger Wolff & Partner
Waidmarkt 11
50676 Köln (DE)**

(56) References cited:
**WO-A1-2020/139952    WO-A1-2020/191150
WO-A1-2020/193615    WO-A1-2021/028130**

**US-A1- 2016 033 463    US-A1- 2021 186 266**

• **UNKNOWN: "Technical Note Contents", 1 October 2014 (2014-10-01), pages 1 - 20, XP093004924, Retrieved from the Internet <URL:https://web.archive.org/web/20220127/ https://www.emerson.com/documents/ automation/ technical-note-guided-wave-radar-interface-mea surement-rosemount-en-78542.pdf> [retrieved on 20221205]**
• **PROF MUNIAPPAN ET AL: "An Overview of Dielectric Properties Measuring Techniques Soil water and carbon sequestration View project Impacting Nutritional Security in India by Scaling up Utilization of Post Harvest and Food Technologies of Small Millets with Mr", 1 January 2005 (2005-01-01), pages 7.15 - 7.30, XP055740130, Retrieved from the Internet <URL:https://www.researchgate.net/profile/ Vijaya_Raghavan5/publication/235435108_An_O verview_of_Dielectric_Properties_Measuring_Te chniques/links/0046353b57fe32f70d000000.pdf> [retrieved on 20201014]**

**EP 4 367 502 B1**

## Description

The present invention relates to the field of frying of food products, in particular on an industrial scale. Specifically, the invention relates to a food processing line comprising a fryer for frying food products comprising a frying vessel for accommodating a frying oil. The invention further relates to a method for frying food products in a frying vessel of a fryer, wherein the frying vessel contains a frying oil or melted frying fat. The invention is defined in the appended claims.

The process of frying includes cooking of food products partly submerged (shallow-frying) or entirely submerged (deep-frying) in a frying oil and/or molten frying fat at high temperatures, in particular in the range of 180 to 190°C. Industry scale fryers typically comprise a conveyor that conveys the food products to be fried through the frying vessel that contains the frying oil. During use, frying oils undergo changes in their chemical composition. In its fresh state, the frying oil does not comprise substantial amounts of polar materials. With continued use of the frying oil, the oil decomposes and the amount of polar materials increases. When the amount of total polar materials (TPM) reaches the range of 24% to 27%, the frying oil is not considered to be suitable for consumption and should therefore be replaced or freshened with replacement frying oil.

The amount of total polar materials can be determined by capacitive measurement, thus, by measuring the capacitance of a capacitor that is submerged in the frying oil. Capacitive measuring instruments for determining TPM in kitchen fryers are available on the market. One of those instruments is the testo 270 cooking oil tester as offered by Testo SE & Co. KGaA.

A similar measurement technique is known from WO 2020/191 150 A1 that discloses a batch fryer for use in a kitchen. An oil caddy is provided that can be attached to the batch fryer to receive oil exiting a frying vat of the batch fryer. The oil caddy includes a sensor for measuring the capacitance of the oil whereas an oil quality is derived from the measured capacitance in terms of total polar materials (TPM) present in the oil.

US 2016/0033463 A1 discloses a fryer that includes a fryer pot, a filter pan connected to the fryer pot by a drain conduit and a return conduit forming a filtration loop, a cooking oil quality sensor being in the filtration loop, and a controller that controls operation of a filtration cycle of the fryer.

In light of the above, it is an object of the present invention to enable determining and controlling the quality of frying oil used in industrial scale fryers.

To better address this concern, in a first aspect of the invention a food processing line according to claim 1 is presented.

According to another aspect of the invention, a method for frying food products in a frying vessel of a fryer according to claim 15 is presented.

The guided wave radar sensor enables determining the permittivity of the frying oil in the frying vessel. Based on the determined permittivity, the quality of the frying oil in the frying vessel may be determined. The guided wave radar sensor further enables in-line real-time determination of permittivity, whereby the processing of food products in industrial scale fryers may be improved.

As compared to conductive and/or capacitive sensors, the guided wave radar sensor not only enables inline measurements but also allows to carry out reliable measurements of permittivity of the frying oil in a wide range of temperatures, e.g., temperatures in the range of 0 °C to 190 °C, in particular in the range of 0 °C to 180 °C. The guided wave radar sensor will not be influenced by dirt floating in the frying oil, e.g., burned crumb.

An optional optical sensor enables determining the colour of the frying oil in the frying vessel or in the at least one storage tank. The colour of the frying oil relates to the visual perception of the human eye which means that the optical sensor is able to detect light in the human visible part of the spectrum. The colour may be determined in the form of a numerical colour value or a combination of multiple colour values or colour components. The optical sensor may be a camera, in particular a digital camera, e.g., a CCD or CMOS camera.

According to the invention, the frying vessel may contain a frying oil. The frying oil may be used for frying the food products in the frying vessel. Alternatively, frying vessel may contain residues or parts of pure water or a composition comprising water and a cleaning agent or a composition comprising water and impurities, e.g., fat, oil or dirt. Water may be accommodated in the frying vessel for cleaning the frying vessel.

According to the method of the invention, the analysis unit may be part of a control system of the fryer and/or may be a unit separate from the control system of the fryer and/or may be part of the control system of a food processing line the fryer is part of and/or may be part of a centralized control system and/or maybe be part of the guided wave radar sensor. The analysis unit may comprise a processor, in particular a programmable processor.

The guided wave radar sensor may comprise a radar transmitter for generating a signal, a waveguide coupled to the radar transmitter for guiding the wave towards and/or through the frying oil and/or a radar receiver for receiving waves reflected by the frying oil and/or a wall of the vessel. The guided wave radar sensor, in particular the waveguide of the guided wave radar sensor, may partially be arranged within the frying oil. The waveguide may be constructed of metal, in particular steel. The waveguide may have the form of a rod or a rope. For example, an end tip of the guided wave radar sensor, in particular the waveguide of the guided wave radar sensor, may be submerged in the frying oil. The guided wave radar sensor is preferably configured to measure time of flight of signals sent by the transmitter over the waveguide and received by the receiver after being reflected by the frying oil and/or a wall of the vessel.

According to a preferred embodiment of the invention, the guided wave radar sensor comprises a stilling well. The stilling well may be implemented as a stilling pipe. The stilling well may surround the waveguide of the guided wave radar sensor so as to reduce influence of disturbances such as flow of the frying oil, impurities in the frying oil, surrounding structures and mechanical impact. Preferably, the stilling well comprises one or more openings through which frying oil may flow from the outside of the stilling well to the inside, and vice versa. The one or more openings may be arranged in a shell surface of the stilling well.

According to a preferred embodiment of the invention, the fryer is configured for continuously frying food products and comprises at least one conveyor for conveying food products to be fried through the frying vessel. The conveyor is preferably configured for conveying food products from a product entry of the fryer to a product exit of the fryer. The conveyer may comprise a belt, in particular an endless belt. The belt may be implemented as a mesh belt so as to allow flow of frying oil through the belt. The fryer may additionally comprise a submerge conveyor that hinders the products from floating to the fluid level of the frying oil. The submerge conveyor may comprise a belt, in particular an endless belt. The belt may be implemented as a mesh belt so as to allow flow of frying oil through the belt. The submerge conveyor is preferably arranged above the conveyor.

According to the invention, the guided wave radar sensor is arranged in the frying vessel. Thereby, the quality of the frying oil may be determined directly at the place the frying process takes place.

According to a particularly preferred embodiment of the invention, the guided wave radar sensor is arranged in a pump chamber which is part of the frying vessel. The pump chamber comprises a pump which serves to circulate frying oil within the fryer. Preferably, the pump chamber and the frying vessel are fluidly connected as communicating vessels so the frying oil at the pump chamber and the frying oil in the frying vessel balance out at the same level.

According to the invention at least one additional guided wave radar sensor is arranged in a storage tank for frying oil. One or more storage tanks can be fluidly connected to the fryer in order to store frying oil. For instance, one storage tank comprises a first type of replacement oil (fresh oil), another storage tank comprises a second type of replacement oil (frying oil which is still usable) and another storage tank comprises frying oil which cannot be used anymore, for instance due to a too high percentage of TPM. Preferably each and every storage tank comprise an additional guided wave radar sensor for determining the permittivity of the frying oil in the respective storage tank and/or an optical sensor for determining the colour of the frying oil. Preferably each and every storage tank comprises valves and/or pumps which are connected to the fryer in order to fill/empty the storage tanks. Preferably, the analysis unit is further configured to determine a permittivity of the frying oil in the respective storage tanks based on measurement data of the additional guided wave radar sensor(s) arranged in the storage tank(s).

According to a further preferred embodiment of the invention at least one additional guided wave radar sensor is arranged in the piping fluidly connecting the frying vessel and the at least one storage tank, preferably multiple storage tanks. By means of the additional guided wave radar sensor in the piping, frying oil can be detected in the piping. This may be helpful in case frying oil is drained from the frying vessel. Thereby, it may be prevented that frying oil remains in the piping after emptying the frying vessel and mixes with cleaning water in a subsequent cleaning step. Further, it may be prevented if cleaning water is removed from the piping before filling the frying vessel with frying oil after the cleaning step has been finished. Thus, unwanted mixing of frying oil and cleaning water can be avoided. The additional guided wave radar sensor arranged in the piping can also be used to determine if cleaning agents remain in the piping, so as to decide if a (further) rinsing step is required to remove all cleaning agents from the piping.

The control unit is configured to control the inflow of replacement frying oil into the frying vessel from the at least one storage tank that is arranged in fluid communication with the frying vessel; and/or the outflow of used frying oil from the frying vessel, preferably to the at least one storage tank that is arranged in fluid communication with the frying vessel additionally based on the measurements of the at least one additional guided wave radar sensor which is arranged in the storage tank.

According to a preferred embodiment of the invention, the analysis unit is configured to determine the permittivity of the frying oil in the vessel based on measurement data of the guided wave radar sensor associated with a signal reflection at an interface between air and the frying oil. The method according to the invention preferably includes that the analysis unit determines the permittivity of the frying oil in the vessel based on measurement data of the guided wave radar sensor associated with a signal reflection at an interface between air and the frying oil. In particular, the permittivity of the frying oil is determined as a function of an amplitude of the signal reflected at the interface between air and frying oil. Preferably, a stronger reflected signal relates to a higher permittivity of the frying oil.

According to a preferred embodiment of the invention, the analysis unit is configured to determine an amount of total polar material of the frying oil in the vessel and/or an amount of free fatty acids in the frying oil in the vessel depending on the determined permittivity, in particular at a certain oil temperature. The method according to the invention preferably includes that the analysis unit determines an amount of total polar material of the frying oil in the vessel and/or an amount of free fatty acids in the frying oil in the vessel depending on the determined permittivity.

According to a preferred embodiment of the invention, the analysis unit is configured to determine the amount of total polar material of the frying oil in the vessel and/or the amount of free fatty acids in the frying oil in the vessel additionally depending on predetermined calibration data. In the method according to the invention, it is preferred that the analysis unit determines the amount of total polar material of the frying oil in the vessel and/or the amount of free fatty acids in the frying oil in the vessel additionally depending on predetermined calibration data. The calibration data preferably includes a relationship of the amount of total polar material of the frying oil in the vessel and/or the amount of free fatty acids in the vessel as a function of permittivity of the frying oil, in particular at a certain oil temperature, and optionally as a function of the type of frying oil. The relationship may be a mathematical function or a look-up table. The calibration data, in particular relating to total polar materials, may be created by measurement using a capacitive sensor. Alternatively, or additionally, the calibration data, in particular relating to free fatty acids, may be created by measurement using test strips or FTIR.

According to a preferred embodiment of the invention, the fryer comprises a temperature sensor for measuring the temperature of the frying oil in the frying vessel, in particular in the pump chamber, wherein the analysis unit is configured to determine the amount of total polar material of the frying oil in the vessel and/or the amount of free fatty acids in the frying oil in the vessel additionally depending on the measured temperature of the frying oil. In the method according to the invention it is preferred that a temperature sensor of the fryer measures the temperature of the frying oil in the frying vessel and the analysis unit determines the amount of total polar material of the frying oil in the vessel and/or the amount of free fatty acids in the frying oil in the vessel additionally depending on the measured temperature of the frying oil. The calibration data may depend on temperature. Thus, the calibration data preferably includes a relationship of the amount of total polar material of the frying oil in the vessel and/or the amount of free fatty acids in the vessel as a function of permittivity of the frying oil and as a function of temperature, and optionally as a function of the type of frying oil.

According to a preferred embodiment of the invention, the analysis unit is configured to determine an amount of foreign matter in the frying vessel, in particular cleaning liquid or water, depending on the determined permittivity. In the method according to the invention, it is preferred that the analysis unit determines an amount of foreign matter in the frying vessel, in particular cleaning liquid or water, depending on the determined permittivity. Thereby, residues of cleaning agents and/or water may be detected in the frying vessel and/or in the frying oil. Depending on the amount of foreign matter in the frying vessel operation of the fryer may be controlled and/or stopped.

According to a preferred embodiment of the invention, the fryer, the food processing line, a centralized control system and/or the guided wave radar sensor comprises a memory unit configured to store a temporal course of the permittivity and/or the amount of total polar material and/or the amount of free fatty acids. The method according to the invention preferably includes that a temporal course of the permittivity and/or the amount of total polar material and/or the amount of free fatty acids is stored. The temporal course of the permittivity and/or the amount of total polar material and/or the amount of free fatty acids may be stored in a logfile, preferable a logfile for each batch of food products to be fried. The logfile may be kept for the purpose of quality control.

According to a preferred embodiment of the invention, the analysis unit is configured to determine a filling level of the frying oil in the frying vessel and/or in a storage tank that is arranged in fluid communication with the frying vessel based on measurements of the respective guided wave radar sensor. In the method according to the invention, it is preferred that the analysis unit determines a filling level of the frying oil in the frying vessel and/or storage tank based on measurements of the respective guided wave radar sensor. The filling level can be determined by measuring the reflection of a signal at the surface of the frying oil, that is the air-oil interface. The analysis unit may calculate the distance between the transmitter and the surface of the frying oil based on the measured time of flight of the reflected signal. According to a preferred embodiment of the invention, the analysis unit is configured to generate a warning if the permittivity exceeds a predetermined threshold and/or if the amount of total polar material exceeds a predetermined threshold and/or if the amount of free fatty acids exceeds a predetermined threshold. In the method according to the invention, it is preferred that a warning is generated if the permittivity exceeds a predetermined threshold and/or the amount of total polar material exceeds a predetermined threshold and/or the amount of free fatty acids exceeds a predetermined threshold. The threshold may, e.g., be in the range of 20% to 22% of total polar materials. The warning can be perceived by a user of the machine, e.g., a machine supervisor who may exchange the frying oil or may add replacement frying oil to the vessel and/or the storage tank of the fryer.

According to the invention, the food processing line comprises a control unit that is configured to control

an inflow of replacement frying oil into the frying vessel, preferably from a storage tank that is arranged in fluid communication with the frying vessel; and/or
an outflow of used frying oil from the frying vessel, preferably to a storage tank that is arranged in fluid communication with the frying vessel
based on the measurements of the guided wave radar sensor.

In the method according to the invention it is foreseen that

an inflow of replacement frying oil into the frying vessel, preferably from a storage tank that is arranged in fluid communication with the frying vessel; and/or

an outflow of used frying oil from the frying vessel, preferably to a storage tank that is arranged in fluid communication with the frying vessel

is controlled based on the measurements of the guided wave radar sensor.

The replacement frying oil may be fresh frying oil or frying oil satisfying certain maximum requirements regarding permittivity and/or total polar materials and/or free fatty acids. Thereby, frying oil quality in the fryer may automatically be adjusted to a predetermined quality range.

For example, the fryer may automatically add replacement frying oil to the vessel from storage tank in case the analysis unit finds that frying oil quality has dropped below a predetermined threshold, e.g., if the amount of total polar materials exceeds a predetermined threshold.

According to a preferred embodiment of the invention, the analysis unit is configured to predict an end of frying oil lifetime at which the permittivity and/or the amount of total polar material and/or the amount of free fatty acids will exceed a predetermined threshold. In the inventive method it is preferred that an end of frying oil lifetime is predicted at which the permittivity and/or the amount of total polar material and/or the amount of free fatty acids will exceed a predetermined threshold. With the predicted end of frying oil lifetime, it is possible to plan ahead exchanging the frying oil and/or refreshing the frying oil, e.g., by adding replacement frying oil to the frying oil in the frying vessel.

According to a preferred embodiment of the invention, the analysis unit is configured to generate a warning based on the measurements of the guided wave radar sensor if the predicted end of lifetime falls within a predetermined time window for processing a batch of products. In the inventive method it is preferred that a warning is generated based on the measurements of the guided wave radar sensor if the predicted end of lifetime falls within a predetermined time window for processing a batch of products. The warning may indicate that it will be required to exchange the frying oil or refresh the frying oil before end of the current batch of food products to be processed.

According to a preferred embodiment of the invention the frying vessel and/or the at least one storage tank comprises a sensor to monitor the oxidation of the frying oil. Preferably, the frying vessel and/or the at least one storage tank comprises a viscosity sensor to measure the viscosity of the frying oil. Preferably, the frying vessel and/or the at least one storage tank comprises a light sensor to monitor the illuminance of the frying oil and/or the polarity of the frying oil. Determining the viscosity and/or the illuminance and/or the polarity may allow to derive the amount of free fatty acids in the frying oil.

According to a preferred embodiment of the invention, the food processing line comprises an external filter which is fluidly connected to the frying vessel. The external filter is preferably a fully automatic, self-cleaning pressure filter, e.g., an Oberlin hot oil filter.

According to a preferred embodiment of the invention, the control unit is further configured to control the external filter based on the measurements of the guided wave radar sensor and/or the optical sensor. Preferably the external filter is controlled by setting an operating parameter and/or an operating property, such as oil pressure, oil flow, velocity of oil, velocity of moving filter means, vacuum settings and residence time.

These and other aspects of the invention will be apparent from and elucidated with reference to the embodiments described hereinafter.

Fig. 1a     depicts a fryer of a food processing line according to an embodiment of the invention in a perspective view;

Fig. 1b     depicts the interior of the fryer shown in Fig 1a;

Fig. 2       depicts a guided wave radar sensor for use in the fryer according Fig. 1;

Fig. 3       depicts another guided wave radar sensor for use in the fryer according Fig. 1;

Fig. 4       depicts a frying oil quality diagram;

Fig. 5       depicts a food processing line according to an embodiment of the invention comprising a fryer and connected storage tanks; and

Fig. 6       depicts a food processing line according to another embodiment of the invention comprising a fryer and an external filter.

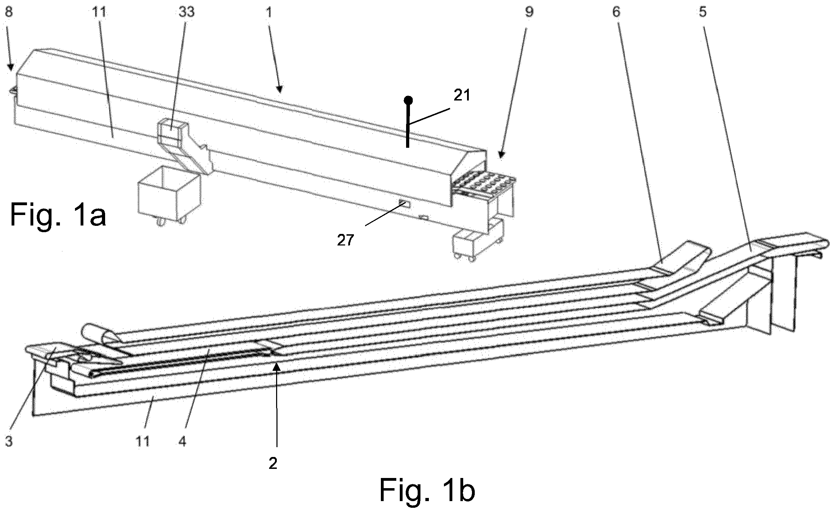

Fig. 1a and 1b illustrate a fryer 1 of a food processing line according to an embodiment of the invention. The fryer 1 is configured as an industry-scale fryer for continuous operation. It comprises a frying vessel 11 and a main conveyor 2 for conveying food products to be fried through the frying vessel 11. The fryer 1 includes a product entry 8 through which the food products enter the fryer 1 during regular operation. On a side opposing the product entry 8, the fryer 1 has a product exit 9 through which food products leave the fryer 1 after having been fried. The main conveyer 2 of the fryer 1 is configured for conveying food products from the product entry 8 to the product exit 9. The main conveyer 4 comprises at least one belt 4, 5, which is implemented as an endless mesh belt. According to the embodiment, the main conveyer 2 includes two belts 4, 5. The fryer 1 may additionally comprise a submerge conveyor 6 that hinders the products from floating to the frying oil surface. The submerge conveyor 6 also has a belt, in particular an endless mesh belt. The submerge conveyor 6 is arranged above the main conveyer 2. The fryer 1 further includes a dirt removal device 33. For controlling operation of the fryer 1, the fryer 1 comprises a control system 27. Alternatively, or additionally, the fryer 1 may be controlled by a control system of a food processing line including the fryer 1 and/or a centralized control system.

In order to determine the quality of the frying oil in the frying vessel 11 the fryer 1 comprises a guided wave radar sensor 21 for determining a permittivity of the frying oil 10 accommodated in the frying vessel 11 based on measurement data of the guided wave radar sensor 21. Based on the permittivity, the quality of the frying oil 10 in the frying vessel 11 may be determined. The guided wave radar sensor 21 further enables in-line real-time determination of frying oil quality, whereby the processing of food products in the industrial scale fryer 1 is improved in case an oil management is enabled.

The fryer 1 according to the embodiment further comprises a temperature sensor 15 for measuring the temperature of the frying oil 10 in the frying vessel 11. The temperature sensor 15 may be submerged in the frying oil 10.

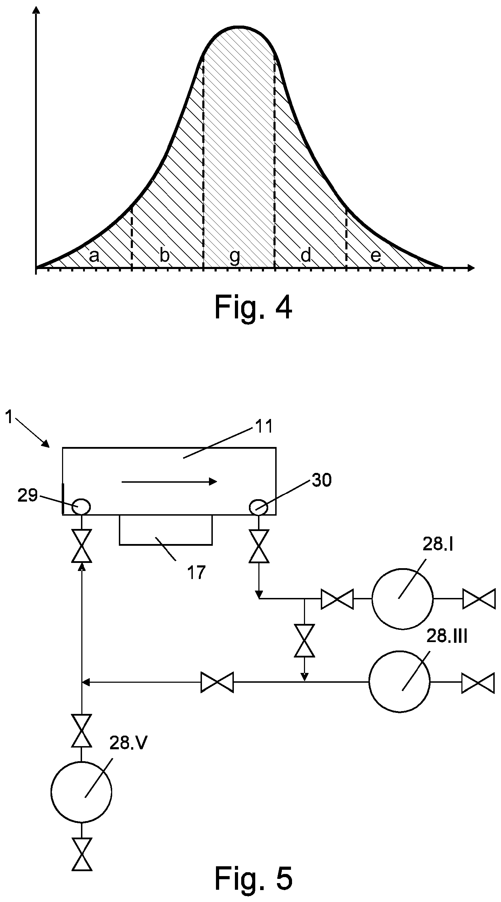

In the fryer 1 depicted in **fig. 1a and 1b,** the guided wave radar sensor 21 is, according to the invention, arranged in the frying vessel 11. However, also according to the invention, an additional guided wave radar sensor 21 (not depicted in **fig. 1a and 1b**) is arranged in a storage tank 28 (depicted in **Fig. 5**) for holding the frying oil which storage tank 28 is fluidly connected to the frying vessel 11.

The fryer 1 may additionally comprise an optical sensor for determining the colour of the frying oil 10 in the frying vessel 11.

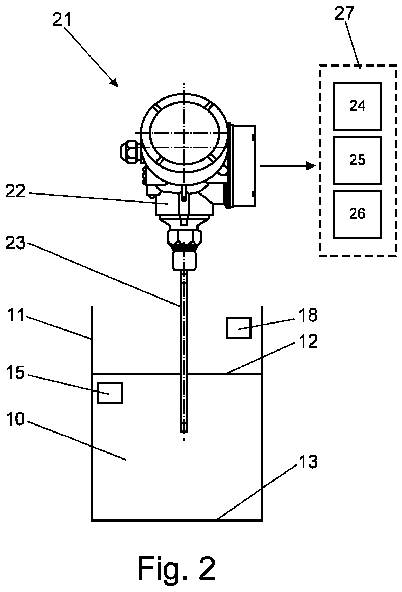

As indicated in **Fig. 2,** the control system 27 of the fryer 1 comprises an analysis unit 24, a control unit 25 and a memory unit 26. The analysis unit 24 of the fryer 1 is configured to determine a permittivity of the frying oil 10 in the vessel 11. Then, the analysis unit 24 determines an amount of total polar material of the frying oil 10 in the vessel 11 and/or an amount of free fatty acids in the frying oil 10 in the vessel 11 based on the determined permittivity and the measured temperature. As the permittivity of frying oil changes during use of the frying oil, measuring the permittivity is a reliable method of determining total polar materials. Together with knowledge about the type of frying oil in the frying vessel 11 it is possible to determine the amount of free fatty acids based on the determined permittivity. The analysis unit 24 uses predetermined calibration data which includes a relationship of the amount of total polar material TPM of the frying oil in the vessel and/or the amount of free fatty acids FFA in the vessel as a function of permittivity of the frying oil and/or temperature, and optionally as a function of the type of frying oil.

The temporal course of the determined values, here the temporal course of the permittivity and/or the amount of total polar material and/or the amount of free fatty acids may be determined and stored in the memory unit 26.

The guided wave radar sensor 21 further enables determining the filling level of the frying oil 10 in the frying vessel 11 and in case a guided wave radar sensor 21 is applied in storage tank 28 also in storage tank 28. Based on the determined filling level, the analysis unit 24 may calculate the amount of frying oil absorbed by the food products.

During operation of the fryer 1, the analysis unit 24 may generate a warning if the permittivity exceeds a predetermined threshold and/or if the amount of total polar material exceeds a predetermined threshold and/or if the amount of free fatty acids exceeds a predetermined threshold. In case a guided wave radar sensor 21 is arranged in the storage tank 28 the warning may be generated with respect to the content of the storage tank 28.

The analysis unit 24 may predict an end of frying oil lifetime at which the permittivity and/or the amount of total polar material and/or the amount of free fatty acids will exceed a predetermined threshold. Then, the analysis unit 24 may generate a warning based on the measurements of the guided wave radar sensor 21 if the predicted end of lifetime falls within a predetermined time window for processing a batch of products. In case a guided wave radar sensor 21 is arranged in the storage tank 28 the warning may be generated with respect to the content of the storage tank 28.

The control unit 25 may control an inflow of replacement frying oil into the frying vessel 11 from the storage tank 28 and/or an outflow of used frying oil 10 from the frying vessel 11 to the storage tank 28 based on the measurements of the guided wave radar sensor 21.

The guided wave radar sensor 21 will be described with reference to **Fig. 2** which depicts an embodiment of a guided wave radar sensor 21 to be employed in a fryer 1 according to the invention.

The guided wave radar sensor 21 comprises a radar transmitter for generating a signal, a waveguide 23 coupled to the radar transmitter for guiding the wave towards and/or through the frying oil 10 and/or a radar receiver for receiving waves reflected by the frying oil 10. In the guided wave radar sensor 21 depicted in Fig. 2, both the transmitter and the receiver are arranged in a common housing 22. The waveguide 23 has the form of a rod or a rope and is partially arranged within the frying oil 10. Here, the free end of the guided wave radar sensor 21, in particular the waveguide 23 of the guided wave radar sensor 21, is submerged in the frying oil 10.

The guided wave radar sensor 21 is configured to measure time of flight of signals sent by the transmitter over the waveguide 23 and received by the receiver after being reflected by the oil surface 12.

According to **Fig. 2,** the food processing line, in particular the fryer 1, includes an optical sensor 18 for determining the colour of the frying oil in the frying vessel 11. The optical sensor 18 is preferably connected with the control system 27, in particular with the control unit 25, so that the control unit 25 may control an inflow of replacement frying oil into the frying vessel 11 and/or an outflow of used frying oil from the frying vessel 11 based on the measurements of the optical sensor 18.

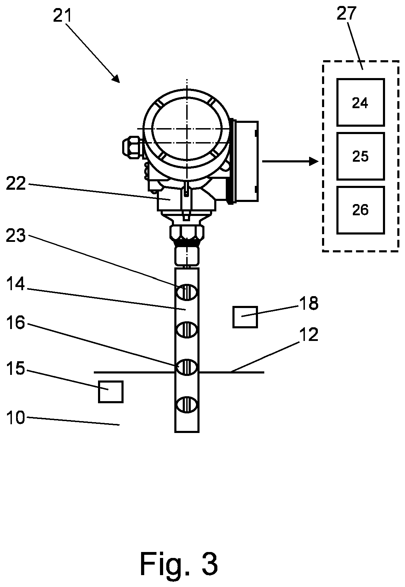

**Fig. 3** depicts an alternative embodiment of a guided wave radar sensor 21 that may be used in the fryer according to the invention. The guided wave radar sensor 21 is similar to the one shown in Fig. 2. In contrast to the guided wave sensor of Fig. 2, the guided wave sensor 21 according to Fig. 3 includes a stilling well 14. The stilling well 14 is arranged coaxial with the waveguide 23. The free end of the stilling well 14 is submerged in the frying oil 10, that means the lower end of the stilling well 14 is located below the air-oil interface 12. The stilling well 14 comprises multiple openings 16 through which frying oil 10 may flow from the outside of the stilling well 14 to the inside, and vice versa. The openings are arranged in a shell surface of the stilling well 14.

**Fig. 4** depicts a frying oil quality diagram. The quality of the frying oil can be classified in five stages:

stage a: Break In (typically 0% to 8% TPM)
stage b: fresh (typically 8% to 14 % TPM)
stage g: optimum (typically 14% to 22 % TPM)
stage d: degrading (typically from 22% to 24% TPM)
stage e: runaway (typically above 24% TPM)

**Fig. 5** depicts an embodiment with multiple storage tanks 28 which are fluidly connected to fryer 1. It is commonly that the fryer vessel 11 and/or pump chamber 17 comprises inflow opening 29 for the supply of replacement oil and outflow opening 30 for oil which will be discharged from fryer 1. In the depicted embodiment inflow oil will be derived from storage tank 28.I (break in/fresh frying oil; stage a) and/or storage tank 28.III (oil with optimum requirements regarding permittivity and/or total polar materials and/or free fatty acids; stage g). Outflow oil will flow towards storage tank 28.V (contaminated oil which no longer can be used; stage e). The number/capacity of fryers and the intended oil management system will determine the number of storage tanks to be used. Valves and pumps will be used in order to manage the oil flows.

The number of storage tanks 28 each with a specific quality of oil is not limited to three or another number of storage tanks 28. In an embodiment with multiple storage tanks the first three storage tanks will be used as described above: A first storage tank 28.I for break-in/new oil, a second storage tank 28.III for optimum oil and a third storage tank 28.V for waste oil. In another embodiment, additionally a fourth storage tank 28.II will be used for fresh oil, this oil has compared to new oil a browner colour. A fifth storage tank 28.IV will be used for degraded oil (oil satisfying certain maximum requirements regarding permittivity and/or total polar materials and/or free fatty acids. This oil has a slightly bad smell. In this embodiment of five oil storage tanks 28 each storage tank represents a stage a or b or g or d or e according Fig. 4, e.g., as summarized below:

stage a: Break In (typically 0% to 8% TPM); oil of yellow/white colour contained in the first storage tank 28.I
stage b: fresh (typically 8% to 14 % TPM); oil of browner colour contained in the fourth storage tank 28.II
stage g: optimum (typically 14% to 22 % TPM); contained in the second storage tank 28.III
stage d: degrading (typically from 22% to 24% TPM); contained in the fifth storage tank 28.IV
stage e: runaway (typically above 24% TPM); contained in the third storage tank 28.V

Optionally, additional storage tanks with a different oil quality and/or oil with a certain colour can be applied.

In the fryer 1 according to the invention, the predetermined threshold for the amount of total polar material is mainly depended by the type of product (for instance the taste, colour) the customer wants to produce, by the local regulations, etcetera and will typically be set to a value in the range of 14% to 26%, preferably 16%-24%, more preferably 20-22%, so that the fryer 1 will operate using frying oil 10 with optimum quality.

In practice the situation can arise that oil from the third storage tank 28.V (used oil; for instance contaminated oil which no longer can be used) will be reused in combination with oil from storage tank 28.IV (oil satisfying certain maximum requirements regarding permittivity and/or total polar materials and/or free fatty acids) and/or with oil from storage tank 28.I (rfor instance, new frying oil). A reason for doing this can be to achieve a certain taste, bite, smell and/or colour (somewhat darker colour) of the fried food products which darker colour will not be achieved when using only new frying oil from the first storage tank 28.I. From importance is that in all cases the quality of the oil used during frying maintains within the desired requirements regarding permittivity and total polar materials and free fatty acids.

If the predetermined threshold is exceeded, replacement frying oil, for instance oil from fourth storage tank 28.II, will be fed into inflow opening 29 within frying vessel 11 from storage tank after used frying oil 10 is drained through outflow opening 30 from frying vessel 11 to third storage tank 28.V in order to lower the amount of total polar materials in the frying oil 10.

As described above, the control system 27 comprises an analysis unit 24, a control unit 25 and a memory 26. If the control unit 25 and the memory 26 are implanted as part of the fryer 1, the analysis unit 24 can also be a unit separate from the fryer 1. The analysis unit 24 can for instance be part of the control system of a food processing line and/or a centralized control system.

In order to operate the fryer 1 and the connected piping and storage tanks 28 in a desired way the control system 27, in particular the control unit 25, implements an oil management system which comprises an oil volume management system and an oil quality management system. The control system 27, in particular the control unit 25, may further implement a cleaning management system in order to control a cleaning procedure, e.g., to be carried out after production.

An oil volume management system is production related and controls the level of frying oil in the fryer 1, knows the design parameters of the fryer (integrated in control system 27) and calculates how much volume of frying oil will be needed to fill the frying vessel 11 up to a desired level. The volume management system also controls the level of frying oil in the oil storage tanks 28, knows the design parameters of the storage tanks (integrated in the control system 27) and calculates how much volume of frying oil is available in the storage tanks 28 and how much volume of frying oil can still be filled in the respective storage tanks 28. In order to determine the filling level of the frying oil in the frying vessel and/or in the storage tanks 28, the analysis unit 24 of the control system 27 receives measurements of the guided wave radar sensors in the frying vessel 11 and/or the storage tanks 28, respectively. Based on those measurements, the inflow of replacement frying oil in to the frying vessel 11 and/or the outflow of used frying oil from the frying vessel 11 is controlled by the control unit 25 of the control system 17. Thereby, unwanted overflow of the fryer and storage tanks can be prevented.

Alternatively, or additionally, another type of fluid level sensor measuring the level of frying oil within the frying vessel 11 and/or the storage tanks 28 can be used which is connected to the oil volume management system implemented in the control system 27.

An oil quality management system is production related and controls parameters related to the correct composition of frying oil used in the fryer 1 such as the desired TPM range, the desired percentage of FFA, the desired colour of the frying oil and the desired smell of the frying oil. Therefor it needs to know the percentage of TPM and/or percentage of FFA and/or the desired colour of the frying oil which is already in the frying vessel 11 of the fryer 1 and of the frying oil available in all the connected storage tanks 28. Preferably, guided wave radar sensors 21 are arranged in the frying vessel 11 and in all storage tanks 28 in order to determine the percentage of TPM of the frying oil in the frying vessel 11 and the storage tanks 28. Optionally, optical sensors 18 are arranged in the frying vessel 11 and all the storage tanks 28, in order to be able to determine the colour of the frying oil within the fryer 1 and the storage tanks 28. Further means to determine the oil quality can be foreseen, as for instance, a sensor to monitor the oxidation of the frying oil and/or sensing means to measure the viscosity of the oil over time (increased viscosity can result in increased oil pick-up). With some types of frying oil, the viscosity correlates with the FFA. Further a light sensor can be used to monitor the illuminance of the oil or the polarity of the oil in order to determine the amount of free fatty acids.

A cleaning management system is related towards the cleaning of the fryer 1 and piping and controls the flow of cleaning water and cleaning agents and performs checks to prevent that cleaning water/agents will mix up with frying oil in order to prevent accidents.

In the following the working principle of the oil management system implemented in the control system 27 will be described using several examples. The examples use five storage tanks 28 in total. The categorization of colour is depicted in the range of 1 to 5. The categorization of TPM, FFA, colour, smell, flavour and taste, however, is not limited to this range. The range could be different for instance, e.g., 1-3-5-7 or 1-2-8-16 in case of four storage tanks, mainly practical results will determine the most suitable and desired range. The algorithm could be provided with an inputted preferred parameter choice. For instance if the colour is from more importance than the TPM value the algorithm will determine the mix of volumes from the different storage tanks such that the colour will be according what is desired.

Example 1: Filling of frying vessel - volume based:

In an embodiment of the invention the empty frying vessel 11 is to be filled with frying oil. The control system 27 calculates how much frying oil is required to achieve a certain level of frying oil in the frying vessel 11. Further the control system 27 selects which storage tanks 28 supply oil towards the fryer and controls the connected valves accordingly. In order to choose which kind of frying oil should be taken from which oil storage tank 28 the control system 27 includes a preferred default sequence. This preferred default sequence can be set by a user via an operating panel or is pre-programmed in the control system 27, in particular in the memory 26. Preferably, the control system 27 is provided with a default minimum level/default volume which should remain in the storage tank 28 for each of the storage tanks 28. Another important parameter is the volume of frying oil which will be supplied via an external supply. In case is known what and how much volume of frying oil will be supplied at a certain date to a certain storage tank the control system can make a reservation for this volume in the specific storage tank. The overall goal is to minimise the use of frying oil and lengthen the time period with which can be worked with the frying oil by optimising the ratio of fresh/used oil. For instance the use of fresh oil in combination with optimum oil will delay the degradation of optimum oil which will result in saving of costs.

Example 2: Filling of frying vessel - TPM/FFA and volume based

In order to fill the frying vessel 11 with frying oil comprising a desired TPM and/or FFA percentage, the oil management system implemented in the control system 27 comprises an algorithm which will calculate, based on the measured TPM and/or FFA percentage in the storage tanks 28, how much frying oil from which storage tank(s) 28 are fed to the frying vessel 11 in order to achieve a composition of mixed frying oil with the desired percentage of TPM and/or FFA and the desired level of oil.

In an embodiment of the invention the percentage of TPM of the frying oil within the frying vessel 11 is controlled within the desired stage, for instance stage "g" as depicted in Fig. 4 between 14%-22%. In calculating the matching volumes of frying oil it is important that the control system 27 takes into account the level/volume of frying oil which is present in each of the multiple storage tanks 28. The calculation is carried out be such that a desired percentage of TPM and colour in the fryer vessel is achieved without running out of frying oil in the storage tanks 28. Further has to be taken in account what the preferred sequence of oil storage tanks 28 is, how much frying oil should remain in each storage tank 28, how much oil will be supplied from external sources towards the specific oil storage tank 28.

An appropriate algorithm is based on the product of the percentage of TPM [-] and the volume of the frying oil [liters]. As an example, storage tank 28.I comprises break-in oil with a TPM of 4%, storage tank 28.III comprises optimum oil with a TPM of 18%, storage tank 28.IV comprises degraded oil with a TPM of 22%. In case the desired percentage of TPM will be 16%±2%, the volume in the fryer will be 1200 liters and the volume in each connected storage tanks is 3500 liters, the algorithm of the oil management system can choose multiple combinations of volumes of frying oil from the connected storage tanks 28.1, 28.111 and 28.IV.

In a first exemplary combination of volumes of oil, 33% oil of storage tank 28.III is preferred. This is (1200 liters * 0.33) = 396 liters. The algorithm to calculate the volumes of frying oil from, in this example, three oil storage tanks:

$$Equation\ A: \left(Volume_{fryer} \cdot TPM_{desired}\right) - \left(Volume_{tank\ 28.III} \cdot 18\right) - \left(Volume_{tank\ 28.I} \cdot 4\right)$$
$$- \left(Volume_{tank\ 28.IV} \cdot 22\right) = 0$$

$$Equation\ A: (1200\ l \cdot 16) - \left(Volume_{tank\ 28.III} \cdot 18\right) - \left(Volume_{tank\ 28.I} \cdot 4\right)$$
$$- \left(Volume_{tank\ 28.IV} \cdot 22\right) = 0$$

$$Equation\ B: Volume_{tank\ 28.I} + Volume_{tank\ 28.IV} = 1200\ l - 396\ l = 804\ l$$
$$=> Volume_{tank\ 28.IV} = 804\ l - Volume_{tank\ 28.I}$$

Combining equation A and B results in a volume of frying oil of storage tank 28.I of 312 liters (26%) and the volume of storage tank 28.IV will be 492 liters (41%) in order to meet the requirement of an overall TPM value of 16.

In a second exemplary combination of volumes of frying oil based on the same algorithm the preferred storage tank to empty is storage tank 28.IV and it is desired to draw only 72 liters of frying oil from storage tank 28.III (6%). The algorithm determines that 384 liters of frying oil (32%) are drawn from storage tank I and 744 liters (62%) from storage tank IV in order to meet the requirement of an overall TPM value of 16.

Example 3: Filling of frying vessel - colour and volume based

In order to fill the frying vessel 11 with frying oil comprising a desired colour, the oil management system implemented in the control system 27 calculates how much frying oil from which storage tank(s) 28 have to be transported towards the fryer 1 in order to achieve the desired colour and the desired level of oil.

In a first embodiment, wherein optical sensor are provided, such as for instance a camera in the fryer 1, the optical sensor monitors the colour gradient of for instance the surface of the oil during filling the frying vessel 11 with frying oil from a specific storage tank 28. The algorithm will determine that for instance, in case the frying vessel 11 is filled for 20% to switch to another storage tank 28 in case the colour differs from the desired colour. This process can be repeated until the frying vessel 11 is filled till the desired level with frying oil of the desired colour.

In a second embodiment, wherein optical sensors are not provided in the frying vessel 11 but in the storage tanks 28 an algorithm determines how much frying oil from which storage tank 28 should be taken in order to end up with the desired colour of oil within the frying vessel 11.

In a third embodiment, optical sensors are provided in the frying vessel 11 as well as in the oil storage tanks 28. The process of filling the frying vessel 11 in case of only an optical sensor 18 is present within the frying vessel 11 can be combined with the algorithm used in case only optical sensors are present within the storage tanks 28. The process of filling the frying vessel 11 of the fryer 1 will be performed and the algorithm related to the storage tanks 28 could be a check-up and intervenes when needed. More preferably the algorithm related to the storage tanks 28 performs the filling of the frying vessel 11 and the optical sensor 18 within the fryer 1 will be the check-up and which will intervene when needed.

In calculating the colour of oil an algorithm could be based on groups of colours wherein each group of colour is represented by a numerical value. For instance, value "1" is assigned to yellow-white oil, value "2" is assigned to a slight brown colour, value "3" is assigned to a golden brown colour, value "4" is assigned to a dark colour and value "5" to a dark-to black colour.

In case there is a relationship between the TPM of the frying oil and the colour of the frying oil a calculation based on volume is sufficient. In an example below the relationship between TPM and colour of the oil and smell is as follows:

Oil storage tank I, TPM=4, Colour yellow-white=1, No smell
Oil storage tank II, TPM=11, Colour slight brown=2
Oil storage tank III, TPM=18, Colour golden brown=3, Appetizing flavour and smell
Oil storage tank IV, TPM=22, Colour dark=4, Slightly bad smell
Oil storage tank V, TPM=25, Colour black=5

In a colour-volume example of a combination of volumes of frying oil for instance 35% oil from storage tank V is preferred. This is (35/100*1200 liter)=420 liter. The desired colour is golden brown, the numeric value is 3. The algorithm to calculate the volumes of oil from in this example three storage tanks:

$$Equation\ C: \left(Volume_{fryer} \cdot colour_{fryer}\right) - \left(Volume_{tank\ I} \cdot 1\right) - \left(Volume_{tank\ II} \cdot 2\right)$$
$$- \left(Volume_{tank\ V} \cdot 5\right) = 0$$

$$Equation\ C: (1200\ l \cdot 3) - \left(Volume_{tank\ I} \cdot 1\right) - \left(Volume_{tank\ II} \cdot 2\right) - (420\ l \cdot 5) = 0$$

$$Equation\ D: Volume_{tank\ I} + Volume_{tank\ II} = 1200\ l - 420\ l = 780\ l$$
$$=> Volume_{tank\ II} = 780\ l - Volume_{tank\ I}$$

Combining equation C and D results in a volume of frying oil from storage tank I of 60 liter (5%) and the volume of storage tank II will be 720 liter (60%) in order to meet the requirement of an overall colour value of 3.

We now refer the second TPM/FFA-volume combination of Example 2 above wherein 72 liters (6%) of frying oil is drawn from storage tankIII, 384 liter oil (32%) drawn from storage tank I and 744 liter (62%) from storage tank IV. The algorithm according based on equation A and C above is able to calculate the colour of the oil in the fryer:

$$Equation\ C: \left(Volume_{fryer} \cdot colour_{fryer}\right) - \left(Volume_{tank\ I} \cdot 1\right) - \left(Volume_{tank\ III} \cdot 3\right)$$
$$- \left(Volume_{tank\ IV} \cdot 4\right) = 0$$

$$Equation\ C: \left(1200\ l \cdot colour_{fryer}\right) - (384\ l \cdot 1) - (72\ l \cdot 3) - (744\ l \cdot 4) = 0$$

As a result of this calculation the resulting colour of the oil in the fryer will be 2.98 which can be defined as golden brown.

In case the percentage of TPM of the frying oil is not representative of the colour (for instance when applying dark oil such as palm oil and/or when processing a product as spinach which will darken the colour of the oil) preferably a calculation based on colour and the percentage of TPM will be done. The TPM value versus the colour differs from the examples above and could for example be:

Oil storage tank I, TPM=4, Colour yellow-white=1
Oil storage tank II, TPM=11, Colour golden brown=2
Oil storage tank III, TPM=18, Colour dark=3
Oil storage tank IV, TPM=22, Colour black=4
Oil storage tank V, TPM=25

Oil storage tank III has still a TPM value of 18 however the colour is dark and therefor possibly not preferable to use. The oil in storage tank 25 is in this example not usable anymore and should be removed. In a TPM/FFA-volume or colour-volume calculation the algorithm and equations A-D described above can be used to come to a mix of oil with an acceptable TPM/FFA value and/or an acceptable colour.

Attention should be taken that oil comprising a strong smell and unwanted flavour, despite an acceptable TMP/FFA value and/or despite an acceptable colour will not be usable anymore.

Example 4: Filling of frying vessel - TPM/FFA and colour and volume based

In order to fill the frying vessel 11 with frying oil comprising the desired TPM and/or FFA percentage and the desired colour, the oil management system implemented in the control system 27 will calculate how much frying oil from which storage tanks 28 will be transported towards the fryer 1 in order to achieve the desired percentage of TPM and/or FFA and the desired colour and the desired level of frying oil.

For instance, from the examples above (TPM/FFA-volume and colour-volume) can be derived:

Oil storage tank I, Volume=384 liter, TPM=4, Colour yellow-white=1, No smell
Oil storage tank III, Volume=72 liter, TPM=18, Colour golden brown=3, Appetizing smell
Oil storage tank IV, Volume=744 liter, TPM=22, Colour dark=4, Slightly bad smell

A mix of these parameters ended up with:
Volume=1200 liter, TPM=16, Colour golden brown=3, Appetizing smell

Therefore, the algorithm comprises a combination of equations (A, B, C, D) and/or the equations will be set-up/merged such that based on similar calculation principles as described in the examples above the algorithm determines the TPM/FFA and colour and volume values at once.

Example 5: Filling of frying vessel - TPM/FFA and colour and smell and flavour and taste and volume based

The algorithms described above can also be used to select frying oil based on a variety of combinations such as volume and TPM and colour and smell, FFA and colour and flavour and smell, volume and TPM and colour and taste, etc. As long as the volumes, TPM, FFA, colour, smell, flavour and taste are categorized as depicted above.

Example 6: Filling of frying vessel - Mixing oil

In an embodiment the oil will be mixed within the frying vessel 11, therefore a circulation system should be introduced to circulate the frying oil within the frying vessel 11. To prevent contact with air the circulation should take place below the oil level surface. In case the fryer vessel is filled above a certain safety level the mixing of oil can be combined with heating de frying oil. The temperature can be controlled by a temperature sensor. In case the oil level is above the outlets of the flow box the flow box can be used to mix the oil or can be used to additionally mix the oil. The oil within the vessel can, depending on the level of the oil in the fryer, be mixed by running the conveyor belts such as a non-stick conveyor, transit conveyor, submerge conveyor, infeed conveyor and takeover conveyor.

In another embodiment mixing of oil outside the oven for instance within a distribution station such that the several types of frying oil are already mixed before and/or during filling the frying vessel.

Example 7: Fryer in production

In case food products are fried during production, the level of oil within the fryer vessel is decreasing mainly due to pick-up of oil by the food products and need to be restored. Therefore the oil management system will control the refilling of the frying vessel 11 until the desired level of oil is reached, this can be achieved under similar conditions and algorithm regarding oil management as described above during filling the fryer, such as determine how much oil from which storage tanks will be transported towards the fryer 1 in order to achieve the desired percentage of TPM/FFA, the desired colour and the desired level of oil. In order to maintain a constant quality of the food products to be fried the quality of the frying oil should be as constant as possible.

Example 8: Emptying of the fryer and the piping

In a further embodiment of the invention the production of food products is finished and the fryer 1 and preferably the connected piping need to be emptied. The oil management system knows the percentage of TPM/FFA of the frying oil in the frying vessel 11 and storage tanks 28, in case the colour of the oil is part of the oil quality management also the colour of the frying oil in the frying vessel 11 and storage tanks 28 and the level of oil within the vessel and the level of oil in the connected storage tanks and based on these parameters oil will be transported from the vessel to the correct storage tank(s).

After emptying the fryer 1 and transporting the frying oil towards the storage tanks 28, the sensor within the piping, preferably a guided wave radar sensor will check the level of oil within the piping to check if there is remaining frying oil. To prevent that this oil will later on mixed up with cleaning water this oil should be removed before introducing water and cleaning agents. Alternatively or additionally another type of fluid level sensor measuring the level of oil within the piping and connected to/controlled by the oil volume management system can be used.

Example 9: Cleaning of the fryer and the piping

In another embodiment of the invention the fryer and preferably the connected piping need to be cleaned. A mix of (hot) water and chemicals clean the machinery, a temperature sensor within the fryer vessel can check if the temperature is sufficient for the cleaning process. After cleaning due to food safety foreign matter and residues of water and cleaning agents should be removed before the fryer vessel will be filled with frying oil in order to prevent that water and/or cleaning agent will be mixed up with the frying oil.

The level of water and/or cleaning agents during cleaning the fryer vessel can be checked preferably by the guided wave radar sensor. After cleaning the fryer, a sensor, preferably the guided wave radar sensor within the fryer should be check if there is still water and/or cleaning agent left behind. This can for instance be done by measuring the level and/or measuring the permittivity to track remaining cleaning fluid/cleaning agents.

Before transporting the oil from the respective storage tanks towards the fryer vessel sensing means positioned in the piping, preferably a guided wave radar sensor, will detect if there is still cleaning water and/or cleaning agents within the piping by measuring an eventual level within the piping and/or measuring the permittivity to track remaining cleaning fluid/cleaning agents. To prevent that cleaning water and/or cleaning agents will enter the fryer it should be removed from the piping.

Alternatively or additionally for both the fryer and the piping another type of sensing means than a guided wave radar sensor will be used for instance a fluid level sensor measuring the level of cleaning water and/or cleaning agents within the piping and connected to/controlled by the oil volume management system can be used. In another embodiment the cleanliness of rinse water will be measured to assure that cleaning agents are not within the piping anymore. In a further embodiment use can be made of concentration measurement to detect a concentrate of chemicals.

Example 10: Smart system - Predictive Forecasting - Remaining lifetime oil:

In an embodiment to assess the remaining lifetime of the frying oil properties such as the percentage of TPM/FFA, colour, smell and taste are from importance. Regarding the percentage of TPM, the course of oil degradation is according a certain pattern; in a graph with vertically the degradation and horizontally the time the degradation will initially follow a slightly rising line and over time this line will be transformed in a curve which will rise sharply in a relative short time. Based on this curve and based on knowing what kind of products will be fried and with which production rate a prediction could be made when a certain batch of oil will need to be replaced based on for instance the percentage of TPM.

By knowing trends in the past and types of mixes of oil used in the past for certain product applications, by knowing the available volumes of oil in the multiple storage tanks, knowing which products will be produced in a defined upcoming period, knowing the upcoming production capacity, knowing the time period a certain type of oil is already used, the oil management system can make a predictive forecast about how much oil with a specific TPM/FFA percentage and colour and smell will be needed and which storage tanks should be filled with oil and/or oil should be removed in order to cover the production in the upcoming period. An alarm message could be sent in case that will be detected that not sufficient oil is available within the factory for the upcoming period and that a certain volume of a certain type of oil should be ordered. To be able to run this predictive forecast system the operating data should be stored in a memory of the control system.

In all examples above there is no limitation towards the numbers of fryers 1 or the number of storage tanks 28.1, 28.11, 28.III, 28.IV and 28.V. Even zero, one or more storage tanks 28.VI, 28.VII, etc. with different composition of oil regarding the percentage of TPM and the desired colour could be part of the oil management system

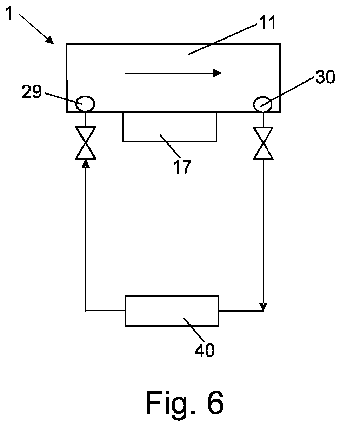

**Fig. 6** depicts another embodiment of a food processing line with a fryer 1 and at least one, preferably multiple, storage tank(s) which are fluidly connected to fryer 1. For better understanding, the storage tank(s) are not depicted in Fig. 6. However, those may be implemented as described in conjunction with Fig. 5. In contrast to the embodiment shown in Fig. 5, the embodiment of Fig. 6 additionally includes an external filter 40 which is fluidly connected to the frying vessel 11 of the fryer 1. By way of the external filter 40, particles may be removed to extend the lifetime of the frying oil. The external filter 40 comes with the benefit that, compared to a filter internal to the frying vessel 11, smaller particles and particles in suspension can be removed. By removing also these smaller particles the food products within the fryer 1 will not come in contact with old particles anymore and therefore frying oil degradation is reduced. For example, the external filter prevents/reduces the change of the percentage of TPM/FFA, colour of the frying oil, smell of the frying oil, flavour of the frying oil and taste of the frying oil.

**EP 4 367 502 B1**

Control unit 27 or the control system of a food processing line or preferably control unit 25 will control the external filter 40 and/or the existing control system of the external filter will be communicatively connected to the control unit 25. The combination of the guided wave radar sensor 21 in the fryer vessel 11 and the external oil filter 40 can further improve the oil quality. In case that for instance the percentage of TPM should be 14%-22% (see Fig. 4) the oil filter can be controlled such that the desired percentage of TPM/FFA could be maintained during production. The operation of the filter is preferably controlled by data derived from the guided wave radar sensor 21. Alternatively or additionally, data measured by the optical sensor 18 can be used to control the external filter 40.

There are different types of external filters 40 available, which may be used in the described embodiment. The different types of external filters 40 use different operating parameters and operating properties such as pressure, oil flow, velocity of oil, velocity of moving filter means, vacuum settings, residence times, etc. Control unit 27 or the control system of a food processing line or the control unit 25 can control the mentioned filter parameters based on data derived from the guided wave radar sensor 21 and/or the optical sensor 18.

The external filter is preferably a fully automatic, self-cleaning pressure filter, e.g., an Oberlin hot oil filter (e.g. the Oberlin G5 OPF7), which filter is able to remove particles with a size greater or equal 1 micron. This filter reduces the breakdown of the frying oil. In general, the smaller the size of the remaining particles in the frying oil, the more the oil quality can be maintained. The used frying oil is pumped towards the external filter device 40 comprising a disposable filter material. The frying oil is pumped through the disposable filter material wherein particles larger as 1 micron are collected and form a cake. The oil pumped through the cake will be transported back to the fryer 1, in particular back to the frying vessel 11. The thicker the formed layer of cake the higher is the pressure drop of the frying oil over the cake. In case a certain pressure drop occurs or in case a certain time period is passed before the pressure drop is reached (to prevent the formed cake from burning), the formed cake is discharged and a new cycle is started with new disposable filter material. The described hot oil filter concerns an almost entire continuous filtration filter (only in case new disposable filter material will be introduced the filtering process is interrupted) wherein particles larger as 1 micron such as crumbs, coating will be removed and discharged automatically as dry cake. Also spices and flavoring will be reduced/removed. Due to the removal of burning/burned particles the forming of a dark oil color will be reduced/prevented. Also, the forming of total polar material TPM and free fatty acids FFA will be reduced/prevented due to the elimination of burning solids.

The control unit 25 can be configured such that the connected external filter 40 is controlled to work more or less intensive in order to manage the use of consumables such as disposable filter materials. In case the external filter 40 is an Oberlin hot oil filter the operating settings are settings which are usually not changed during production. As described above the parameters to adjust will be the pressure drop of oil over the filter/cake and the time period before applying new disposable filter material. Further, the flow of frying oil from the fryer 1 towards the external filter 40 could be controlled.

In an embodiment of the invention the external filter 40 will be monitored and preferably controlled by the control unit 25. In case that within the external filter 40 the build-up of cake during production is for instance at a certain moment quite slow, the control unit 25 can extend the time before new disposable material will be applied. In case the build-up of cake increases the control unit 25 can reduce the time before new disposable material will be applied.

In another embodiment, the flow of frying oil from the fryer 1 towards the external filter 40 can be adjusted depending on the number of particles within the fryer 1. In case there is an accumulation of particles within the fryer 1 the external filter 40 should work more intensively which could be achieved by transporting more frying oil from the fryer 1 towards the external filter 40. Monitoring of the number of particles can be done by monitoring the external filter 40. If the external filter 40 is an Oberlin filter the time wherein a certain pressure drop over the cake exists is a measurement of the number of particles in the fryer 1. In another embodiment the optical sensor 18, such as a camera, can be used at least to monitor/measure the floating particles on the oil surface within the vessel 11 and subsequently to control the flow of frying oil towards the external filter 40 and/or control the parameters of the external filter 40. Optionally, sensing means such as load cells can be used which weigh the bin that collects the sediment (such as larger meat and/or coating particles) and/or floating particles. In case the weight of the bin reaches a certain weight within a predetermined time period the control unit 25 determines the degree of pollution and will decide if the external filter 40 should work more intensively.

List of reference signs:

| | |
|---|---|
| 1 | fryer |
| 2 | main conveyor |
| 3 | infeed conveyor |
| 4 | belt |
| 5 | belt |
| 6 | submerge conveyor |
| 8 | food product entry |

13

| 9 | food product exit |
|---|---|
| 10 | frying oil |
| 11 | frying vessel |
| 12 | air-oil-interface |
| 13 | bottom |
| 14 | stilling well |
| 15 | temperature sensor |
| 16 | opening |
| 17 | pump chamber |
| 18 | optical sensor |
| 21 | guided wave radar sensor |
| 22 | housing |
| 23 | waveguide |
| 24 | analysis unit |
| 25 | control unit |
| 26 | memory unit |
| 27 | control system |
| 28,28I-III | storage tank |
| 29 | oil inflow opening |
| 30 | oil outflow opening |
| 33 | dirt removal device |
| 40 | external filter |

**Claims**

1. Food processing line comprising a fryer (1) for frying food products comprising

    a frying vessel (11) for accommodating a frying oil (10),

        the food processing line further comprising at least one storage tank (28.1, 28.111, 28.V) arranged in fluid communication with the frying vessel (11),
        the food processing line further comprising

    a first guided wave radar sensor (21) arranged in the frying vessel (11) and
    an analysis unit (24) for determining a permittivity of the frying oil (10) accommodated in the frying vessel (11) based on measurement data of the guided wave radar sensor (21)
    and at least one additional guided wave radar sensor is arranged in the at least one storage tank (28.1, 28.III, 28.V),
    the food processing line further comprising a control unit (25) that is configured to control
    an inflow of replacement frying oil into the frying vessel (11) from the at least one storage tank (28.I, 28.III, 28.V) that is arranged in fluid communication with the frying vessel (11); and/or
    an outflow of used frying oil from the frying vessel (11) to the at least one storage tank (28.I, 28.III, 28.V) that is arranged in fluid communication with the frying vessel (11)
    based on the measurements of the first guided wave radar sensor (21) and based on the measurements of the at least one additional guided wave radar sensor which is arranged in the storage tank (28.1, 28.III, 28.V) for frying oil.

2. Food processing line according to claim 1, **characterized by** an optical sensor (18) for determining the colour of the frying oil (10) in the frying vessel (11) or in the at least one storage tank (28.1, 28. III, 28.V), wherein the control unit (25) is configured to control

    an inflow of replacement frying oil into the frying vessel (11), preferably from the at least one storage tank (28.1, 28.111, 28.V) that is arranged in fluid communication with the frying vessel (11); and/or
    an outflow of used frying oil from the frying vessel (11), preferably to the at least one storage tank (28.I, 28.III, 28.V) that is arranged in fluid communication with the frying vessel (11)
    additionally based on the measurements of the optical sensor (18).

3. Food processing line according to any of the preceding claims, **characterized in that** the fryer (1) is configured for continuously frying food products and comprises at least one conveyor (2) for conveying food products to be fried through the frying vessel (11).

4. Food processing line according to any of the preceding claims, **characterized in that** the fryer (1) comprises a pump chamber (17) which is part of the frying vessel (11) and the first guided wave radar sensor (21) is arranged in the pump chamber (17).

5. Food processing line according to any of the preceding claims, **characterized by** a first storage tank (28.I) that comprises a first type of replacement oil, in particular fresh oil, a second storage tank (28.III) that comprises a second type of replacement oil, in particular frying oil which is still usable, and a third storage tank (28.V) that comprises a third type of replacement oil which cannot be used anymore.

6. Food processing line according to any of the preceding claims, **characterized in that** the analysis unit is configured to determine the permittivity of the frying oil (10) in the vessel (11) based on measurement data of the first guided wave radar sensor (21) associated with a signal reflection at an interface between air and the frying oil (10).

7. Food processing line according to any of the preceding claims, **characterized in that** the analysis unit is configured to determine an amount of total polar material of the frying oil (10) in the vessel (11) and/or an amount of free fatty acids in the frying oil (10) in the vessel (11) depending on the determined permittivity.

8. Food processing line according to claim 7 **characterized in that** the analysis unit is configured to determine the amount of total polar material of the frying oil (10) in the vessel (11) and/or the amount of free fatty acids in the frying oil (10) in the vessel (11) additionally depending on predetermined calibration data.

9. Food processing line according to claim 7 or 8 **characterized in that** the fryer (1) comprises a temperature sensor for measuring the temperature of the frying oil (11) in the frying vessel (11), wherein the analysis unit is configured to determine the amount of total polar material of the frying oil (10) in the vessel (11) and/or the amount of free fatty acids in the frying oil (10) in the vessel (11) additionally depending on the measured temperature of the frying oil (11).

10. Food processing line according to any of the preceding claims, **characterized in that** the analysis unit is configured to determine an amount of foreign matter in the frying vessel (11), in particular cleaning liquid or water, depending on the determined permittivity.

11. Food processing line according to any of the preceding claims, **characterized by** a memory unit configured to store a temporal course of the permittivity and/or the amount of total polar material and/or the amount of free fatty acids.

12. Food processing line according to any of the preceding claims, **characterized in that** the analysis unit is configured to determine a filling level of the frying oil (10) in the frying vessel (11) and/or in a storage tank that is arranged in fluid communication with the frying vessel (11) based on measurements of the first guided wave radar sensor (21).

13. Food processing line according to any of the preceding claims, **characterized in that** the analysis unit is configured to generate a warning if the permittivity exceeds a predetermined threshold and/or if the amount of total polar material exceeds a predetermined threshold and/or if the amount of free fatty acids exceeds a predetermined threshold.

14. Food processing line according to any of the preceding claims, **characterized in that** the analysis unit is configured to predict an end of frying oil lifetime at which the permittivity will exceed a predetermined threshold.

15. Method for frying food products in a frying vessel (11) of a fryer (1), wherein the frying vessel (11) contains frying oil (10) or melted frying fat, in particular wherein at least one conveyor (2) conveys the food products to be fried through the frying vessel (11), and wherein at least one storage tank (28.1, 28.III, 28.V) is arranged in fluid communication with the frying vessel (11),

**characterized in that** the method comprises the following method steps:

an analysis unit determines a permittivity of the frying oil (10) accommodated in the frying vessel (11) based on measurement data of a first guided wave radar sensor (21) arranged in the frying vessel (11) and the permittivity of the frying oil accommodated in the storage tank (28.I, 28.III, 28.V) based on measurement of at least one additional guided wave radar sensor that is arranged in the at least one storage tank (28.I, 28.III, 28.V);
wherein the method further comprises controlling
an inflow of replacement frying oil into the frying vessel (11), preferably from the at least one storage tank (28.1, 28.111, 28.V) that is arranged in fluid communication with the frying vessel (11); and/or
an outflow of used frying oil from the frying vessel (11), preferably to the at least one storage tank (28.I, 28.III, 28.V) that is arranged in fluid communication with the frying vessel (11)

based on the measurements of the first guided wave radar sensor (21) and based on the measurements of the at least one additional guided wave radar sensor that is arranged in the at least one storage tank (28.1, 28.111, 28.V) for frying oil.

**Patentansprüche**

1. Lebensmittelverarbeitungslinie mit einer Fritteuse (1) zum Frittieren von Lebensmitteln, die einen Frittierbehälter (11) zur Aufnahme von Frittieröl (10) aufweist,

   wobei die Lebensmittelverarbeitungslinie außerdem mindestens einen Vorratsbehälter (28.I, 28.III, 28.V) umfasst, der in Fluidverbindung mit dem Frittierbehälter (11) steht, wobei die Lebensmittelverarbeitungslinie außerdem umfasst

   einen ersten Geführte-Wellen-Radarsensor (21), der in dem Frittierbehälter (11) angeordnet ist, und eine Analyseeinheit (24) zum Bestimmen einer Permittivität des in dem Frittierbehälter (11) befindlichen Frittieröls (10) basierend auf den Messdaten des Geführte-Wellen-Radarsensors (21) und in dem mindestens einen Vorratsbehälter (28.I, 28.III, 28.V) mindestens ein zusätzlicher Geführte-Wellen-Radarsensor angeordnet ist,

   wobei die Lebensmittelverarbeitungslinie außerdem eine Steuereinheit (25) umfasst, die dazu konfiguriert ist,

   einen Zufluss von Ersatzfrittieröl in den Frittierbehälter (11) aus dem mindestens einen Vorratsbehälter (28.I, 28.III, 28.V) zu steuern, der in Fluidverbindung mit dem Frittierbehälter (11) angeordnet ist; und/oder einen Abfluss von gebrauchtem Frittieröl aus dem Frittierbehälter (11) in mindestens einen Vorratsbehälter (28.I, 28.III, 28.V), der in Fluidverbindung mit dem Frittierbehälter (11) angeordnet ist auf der Grundlage der Messungen des ersten Geführte-Wellen-Radarsensors (21) und auf der Grundlage der Messungen des mindestens einen zusätzlichen Geführte-Wellen-Radarsensors, der in dem Vorratsbehälter (28.I, 28.III, 28.V) für Frittieröl angeordnet ist.

2. Lebensmittelverarbeitungslinie nach Anspruch 1, **gekennzeichnet durch** einen optischen Sensor (18) zur Bestimmung der Farbe des Frittieröls (10) im Frittierbehälter (11) oder in dem mindestens einen Vorratsbehälter (28.I, 28.III, 28.V), wobei die Steuereinheit (25) konfiguriert ist zum Steuern

   eines Zuflusses von Ersatz-Frittieröl in den Frittierbehälter (11), vorzugsweise aus dem mindestens einen Vorratsbehälter (28.1, 28.III, 28.V), der in Fluidverbindung mit dem Frittierbehälter (11) angeordnet ist; und/oder eines Abflusses von gebrauchtem Frittieröl aus dem Frittierbehälter (11), vorzugsweise zu dem mindestens einen Vorratsbehälter (28.I, 28.III, 28.V), der in Fluidverbindung mit dem Frittierbehälter (11) angeordnet ist, zusätzlich auf der Grundlage der Messungen des optischen Sensors (18).

3. Lebensmittelverarbeitungslinie nach einem der vorhergehenden Ansprüche, **dadurch gekennzeichnet, dass** die Fritteuse (1) zum kontinuierlichen Frittieren von Lebensmitteln ausgebildet ist und mindestens einen Förderer (2) zum Fördern von zu frittierenden Lebensmitteln durch den Frittierbehälter (11) aufweist.

4. Lebensmittelverarbeitungslinie nach einem der vorhergehenden Ansprüche, **dadurch gekennzeichnet, dass** die Fritteuse (1) eine Pumpenkammer (17) aufweist, die Teil des Frittierbehälters (11) ist, und der erste Geführte-Wellen-Radarsensor (21) in der Pumpenkammer (17) angeordnet ist.

5. Lebensmittelverarbeitungslinie nach einem der vorhergehenden Ansprüche, **gekennzeichnet durch** einen ersten Vorratstank (28.1), der eine erste Art von Ersatzöl, insbesondere frisches Öl, enthält, einen zweiten Vorratstank (28.III), der eine zweite Art von Ersatzöl, insbesondere noch verwendbares Frittieröl, enthält, und einen dritten Vorratstank (28.V), der eine dritte Art von Ersatzöl enthält, das nicht mehr verwendet werden kann.

6.  Lebensmittelverarbeitungslinie nach einem der vorhergehenden Ansprüche, **dadurch gekennzeichnet, dass** die Analyseeinheit dazu konfiguriert ist, die Permittivität des Frittieröls (10) in dem Behälter (11) auf der Grundlage von Messdaten des ersten Geführte-Wellen-Radarsensors (21) zu bestimmen, die mit einer Signalreflexion an einer Grenzfläche zwischen Luft und dem Frittieröl (10) verbunden sind.

7.  Lebensmittelverarbeitungslinie nach einem der vorhergehenden Ansprüche, **dadurch gekennzeichnet, dass** die Analyseeinheit dazu konfiguriert ist, eine Menge des gesamten polaren Materials des Frittieröls (10) in dem Behälter (11) und/oder eine Menge freier Fettsäuren in dem Frittieröl (10) in dem Behälter (11) in Abhängigkeit von der ermittelten Permittivität zu bestimmen.

8.  Lebensmittelverarbeitungslinie nach Anspruch 7 **dadurch gekennzeichnet, dass** die Analyseeinheit dazu konfiguriert ist, die Menge des gesamten polaren Materials des Frittieröls (10) im Behälter (11) und/oder die Menge der freien Fettsäuren im Frittieröl (10) in dem Behälter (11) zusätzlich abhängig von vorgegebenen Kalibrierdaten zu bestimmen.

9.  Lebensmittelverarbeitungslinie nach Anspruch 7 oder 8, **dadurch gekennzeichnet, dass** die Fritteuse (1) einen Temperatursensor zur Messung der Temperatur des Frittieröls (11) im Frittierbehälter (11) umfasst, wobei die Analyseeinheit dazu konfiguriert ist, die Menge des gesamten polaren Materials des Frittieröls (10) im Behälter (11) und/oder die Menge der freien Fettsäuren im Frittieröl (10) im Behälter (11) zusätzlich abhängig von der gemessene Temperatur des Frittieröls (11) zu bestimmen.

10. Lebensmittelverarbeitungslinie nach einem der vorhergehenden Ansprüche, **dadurch gekennzeichnet, dass** die Analyseeinheit dazu ausgebildet ist, in Abhängigkeit von der ermittelten Permittivität eine Menge an Fremdstoffen im Frittiergefäß (11), insbesondere Reinigungsflüssigkeit oder Wasser, zu bestimmen.

11. Lebensmittelverarbeitungslinie nach einem der vorhergehenden Ansprüche, **gekennzeichnet durch** eine Speichereinheit, die so konfiguriert ist, dass sie einen zeitlichen Verlauf der Permittivität und/oder der Menge des gesamten polaren Materials und/oder der Menge der freien Fettsäuren speichert.

12. Lebensmittelverarbeitungslinie nach einem der vorhergehenden Ansprüche, **dadurch gekennzeichnet, dass** die Analyseeinheit dazu konfiguriert ist, einen Füllstand des Frittieröls (10) in dem Frittierbehälter (11) und/oder in einem Vorratsbehälter, der in Fluidverbindung mit dem Frittierbehälter (11) angeordnet ist, basierend auf Messungen des ersten Geführte-Wellen-Radarsensors (21) zu bestimmen.

13. Lebensmittelverarbeitungslinie nach einem der vorhergehenden Ansprüche, **dadurch gekennzeichnet, dass** die Analyseeinheit dazu konfiguriert ist, eine Warnung zu erzeugen, wenn die Permittivität einen vorbestimmten Schwellenwert überschreitet und/oder wenn die Menge des gesamten polaren Materials einen vorbestimmten Schwellenwert überschreitet und/oder wenn die Menge der freien Fettsäuren einen vorbestimmten Schwellenwert überschreitet.

14. Lebensmittelverarbeitungslinie nach einem der vorangehenden Ansprüche, **dadurch gekennzeichnet, dass** die Analyseeinheit dazu konfiguriert ist, ein Ende der Lebensdauer des Frittieröls vorherzusagen, bei dem die Permittivität einen vorbestimmten Schwellenwert überschreiten wird.

15. Verfahren zum Frittieren von in einem Frittierbehälter (11) einer Fritteuse (1), wobei der Frittierbehälter (11) Frittieröl (10) oder geschmolzenes Frittierfett enthält, insbesondere wobei mindestens ein Förderer (2) die zu frittierenden Lebensmittel durch den Frittierbehälter (11) fördert, und wobei mindestens ein Vorratsbehälter (28.1, 28.III, 28.V) in Fluidverbindung mit dem Frittierbehälter (11) angeordnet ist,

    **dadurch gekennzeichnet, dass** das Verfahren die folgenden Verfahrensschritte umfasst:
    eine Analyseeinheit bestimmt eine Permittivität des im Frittierbehälter (11) befindlichen Frittieröls (10) auf der Grundlage von Messdaten eines ersten Geführte-Wellen-Radarsensors (21) im Frittierbehälter (11) und eine Permittivität des in dem Vorratsbehälter (28.I, 28.III, 28.V) befindlichen Frittieröls auf der Grundlage einer Messung mindestens eines zusätzlichen Geführte-Wellen-Radarsensors, der in dem mindestens einen Vorratsbehälter (28.I, 28.III, 28.V) angeordnet ist;
    wobei das Verfahren ferner aufweist die Steuerung

EP 4 367 502 B1

eines Zuflusses von Ersatzfrittieröl in den Frittierbehälter (11), vorzugsweise aus dem mindestens einen Vorratsbehälter (28.1, 28.III, 28.V), der in Fluidverbindung mit dem Frittierbehälter (11) angeordnet ist; und/oder

eines Abflusses von verbrauchtem Frittieröl aus dem Frittierbehälter (11), vorzugsweise zu dem mindestens einen Vorratsbehälter (28.I, 28.III, 28.V), der in Fluidverbindung mit dem Frittierbehälter (11) angeordnet ist

auf der Grundlage der Messungen des ersten Geführte-Wellen-Radarsensors (21) und auf der Grundlage der Messungen des mindestens einen zusätzlichen Geführte-Wellen-Radarsensors, der in dem mindestens einen Vorratsbehälter (28.I, 28.III, 28.V) für Frittieröl angeordnet ist.

**Revendications**

1. Ligne de traitement des aliments comprenant une friteuse (1) pour la friture de produits alimentaires comprenant un récipient de friture (11) destiné à recevoir une huile de friture (10),

   selon lequel la ligne de traitement des aliments comprend en outre au moins un réservoir de stockage (28.I, 28.III, 28.V) disposé en communication fluide avec le récipient de friture (11),
   selon lequel la ligne de transformation des aliments comprend en outre

   un premier capteur radar à ondes guidées (21) placé dans le récipient de friture (11) et
   une unité d'analyse (24) pour déterminer la permittivité de l'huile de friture (10) contenue dans le récipient de friture (11) sur la base des données de mesure du capteur radar à ondes guidées (21)
   et au moins un capteur radar à ondes guidées supplémentaire est placé dans ledit au moins un réservoir de stockage (28.I, 28.III, 28.V),

   selon lequel ligne de traitement des aliments comprend en outre une unité de commande (25) configurée pour contrôler

   un afflux d'huile de friture de remplacement dans le récipient de friture (11) à partir dudit au moins un réservoir de stockage (28.I, 28.III, 28.V) disposé en communication fluidique avec le récipient de friture (11) ; et/ou
   un écoulement de l'huile de friture usagée de le récipient de friture (11) vers ledit au moins un réservoir de stockage (28.I, 28.III, 28.V) disposé en communication fluidique avec le récipient de friture (11)
   sur la base des mesures du premier capteur radar à ondes guidées (21) et sur la base des mesures dudit au moins un capteur radar à ondes guidées supplémentaire placé dans réservoir de stockage (28.I, 28.III, 28.V) de l'huile de friture.

2. Ligne de traitement des aliments selon la revendication 1, **caractérisée par** un capteur optique (18) pour déterminer couleur de l'huile de friture (10) dans le récipient de friture (11) ou dans ledit au moins un réservoir de stockage (28.I, 28. III, 28.V), dans laquelle l'unité de commande (25) est configurée pour contrôler

   un afflux d'huile de friture de remplacement dans le récipient de friture (11), de préférence à partir du réservoir de stockage (28.I, 28.III, 28.V), disposé en communication fluidique avec le récipient de friture (11); et/ou
   un écoulement de l'huile de friture usagée depuis le récipient de friture (11), de préférence vers au moins un réservoir de stockage (28.I, 28.III, 28.V) disposé en communication fluidique avec le récipient de friture (11)
   en outre sur la base des mesures du capteur optique (18).

3. Ligne de traitement des aliments selon l'une des revendications précédentes, **caractérisée en ce que** la friteuse (1) est configurée pour frire des produits alimentaires en continu et comprend au moins un convoyeur (2) pour transporter les produits alimentaires à frire à travers le récipient de friture (11).

4. Ligne de traitement des aliments selon l'une des revendications précédentes, **caractérisée en ce que** la friteuse (1) comprend une chambre de pompage (17) qui fait partie de le récipient de friture (11) et le premier capteur radar à ondes guidées (21) est placé dans chambre de pompage (17).

5. Ligne de traitement des aliments selon l'une des revendications précédentes, **caractérisée en ce qu'**un premier réservoir de stockage (28.1) qui contient un premier type d'huile de remplacement, en particulier de l'huile fraîche, un deuxième réservoir de stockage (28.III) qui contient un deuxième type d'huile de remplacement, en particulier de l'huile de friture qui est encore utilisable, et un troisième réservoir de stockage (28.V) qui contient un troisième type d'huile de remplacement qui ne peut plus être utilisé.

6. Ligne de traitement des aliments selon l'une des revendications précédentes, **caractérisée en ce que** l'unité d'analyse est configurée pour déterminer la permittivité de l'huile de friture (10) dans le récipient (11) sur la base des données de mesure du premier capteur radar à ondes guidées (21) associées à une réflexion du signal à une interface entre l'air et l'huile de friture (10).

7. Ligne de traitement des aliments selon l'une des revendications précédentes, **caractérisée en ce que** l'unité d'analyse est configurée pour déterminer une quantité de matière polaire totale de l'huile de friture (10) dans le récipient (11) et/ou une quantité d'acides gras libres dans l'huile de friture (10) dans le récipient (11) en fonction de la permittivité déterminée.

8. Ligne de traitement des aliments selon revendication 7, **caractérisée en ce que** l'unité d'analyse est configurée pour déterminer la quantité de matière polaire totale de l'huile de friture (10) dans le récipient (11) et/ou la quantité d'acides gras libres dans l'huile de friture (10) dans le récipient (11) en outre en fonction de données d'étalonnage prédéterminées.

9. Ligne de traitement des aliments selon revendication 7 ou 8 **caractérisée en ce que** la friteuse (1) comprend un capteur de température pour mesurer la température de l'huile de friture (11) dans le récipient de friture (11), l'unité d'analyse étant configurée pour déterminer la quantité de matière polaire totale de l'huile de friture (10) dans le récipient (11) et/ou la quantité d'acides gras libres dans l'huile de friture (10) dans le récipient (11) en outre en fonction de la température mesurée de l'huile de friture (11).

10. Ligne de traitement des aliments selon l'une des revendications précédentes, **caractérisée en ce que** l'unité d'analyse est configurée pour déterminer une quantité de corps étrangers dans le récipient de friture (11), en particulier un liquide de nettoyage ou de l'eau, en fonction de la permittivité déterminée.

11. Ligne de traitement des aliments selon l'une des revendications précédentes, **caractérisée par** une unité de mémoire configurée pour stocker une évolution temporelle de la permittivité et/ou de la quantité de matière polaire totale et/ou de la quantité d'acides gras libres.

12. Ligne de traitement des aliments selon l'une des revendications précédentes, **caractérisée en ce que** l'unité d'analyse est configurée pour déterminer un niveau de remplissage de l'huile de friture (10) dans le récipient de friture (11) et/ou dans un réservoir de stockage disposé en communication fluide avec le récipient de friture (11) sur la base des mesures du premier capteur radar à ondes guidées (21).

13. Ligne de traitement des aliments selon l'une des revendications précédentes, **caractérisée en ce que** l'unité d'analyse est configurée pour générer une alerte si la permittivité dépasse une valeur seuil prédéterminée et/ou si la quantité de matière polaire totale dépasse une valeur seuil prédéterminée et/ou si la quantité d'acides gras libres dépasse une valeur seuil prédéterminé et/ou si la quantité d'acides gras libres dépasse un seuil prédéterminée.

14. Ligne de traitement des aliments selon l'une des revendications précédentes, **caractérisée en ce que** l'unité d'analyse est configurée pour prédire une fin de vie de l'huile de friture à laquelle la permittivité dépassera une valeur seuil prédéterminée.

15. Méthode de friture de produits alimentaires dans un récipient de friture (11) d'une friteuse (1), dans lequel le récipient de friture (11) contient de l'huile de friture (10) ou de la graisse de friture fondue, en particulier dans lequel au moins un convoyeur (2) transporte les produits alimentaires à frire à travers le récipient de friture (11), et dans lequel au moins un réservoir de stockage (28.1, 28.III, 28.V) est arrangé en communication fluidique avec le récipient de friture (11),

   **caractérisée en ce que** la méthode comprend les étapes suivantes :

   une unité d'analyse détermine la permittivité de l'huile de friture (10) contenue dans le récipient de friture (11) sur la base des données de mesure d'un premier capteur radar à ondes guidées (21) disposée dans le récipient de friture (11) et la permittivité de l'huile de friture dans le réservoir de stockage (28.I, 28.III, 28.V) sur la base de la mesure dudit au moins un capteur radar à ondes guidées supplémentaire placé dans ledit au moins réservoir de stockage (28.I, 28.III, 28.V) ;

dans laquelle la méthode comprend en outre le contrôle

d'un afflux d'huile de friture de remplacement dans le récipient de friture (11), de préférence à partir dudit au moins un réservoir de stockage (28.1, 28.III, 28.V) disposé en communication fluide avec le récipient de friture (11) ; et/ou

d'un écoulement de l'huile de friture usagée depuis le récipient de friture (11), de préférence vers au moins un réservoir de stockage (28.I, 28.III, 28.V) qui est disposé en communication fluidique avec le récipient de friture (11)

sur la base des mesures du premier capteur radar à ondes guidées (21) et sur la base des mesures dudit au moins un capteur radar à ondes guidées supplémentaire qui est disposé dans au moins un réservoir de stockage (28.I, 28.III, 28.V) pour l'huile de friture.

Fig. 1a

Fig. 1b

Fig. 2

Fig. 3

Fig. 4

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020191150 A1
- US 20160033463 A1